# EUROPEAN PATENT SPECIFICATION

(11) **EP 3 339 857 B1**
(45) Date of publication and mention of the grant of the patent: **14.12.2022**
(21) Application number: 16461578.3
(22) Date of filing: 20.12.2016
(51) Int. Cl.: G01N 33/22, F02C 9/00

(54) **SYSTEM AND METHOD FOR POWER-PLANT FUEL QUALITY MEASUREMENT**
SYSTEM UND VERFAHREN ZUR MESSUNG DER QUALITÄT VON KRAFTWERKSBRENNSTOFF
SYSTÈME ET PROCÉDÉ DE MESURE DE LA QUALITÉ DU CARBURANT D'UNE CENTRALE ÉNERGÉTIQUE

(43) Date of publication of application: 27.06.2018
(73) Proprietor: General Electric Company, Schenectady, NY 12345 (US)
(72) Inventor: Dinu, Constantin, Greenville, SC 29615 (US); Dinari, Otman, Greenville, SC 29615 (US); Knapczyk, Michal, 02-256 Warszawa (PL)
(74) Representative: BRP Renaud & Partner mbB Rechtsanwälte Patentanwälte Steuerberater

(56) References cited:
- AU-A4- 2012 100 395
- US-A1- 2009 043 415
- US-A1- 2016 283 254
- DATABASE WPI Week 200531 Thomson Scientific, London, GB; AN 2005-298617 XP002771255, & JP 2005 083255 A (HITACHI LTD) 31 March 2005 (2005-03-31)
- ARUN. P.R ET AL: "Monitoring of Fuel Supply in Power Plant Boilers using LabVIEW", INTERNATIONAL JOURNAL OF ADVANCED RESEARCH IN ELECTRICAL ELECTRONICS AND INSTRUMENTATION ENGINEERING, vol. 03, no. 09, 20 September 2014 (2014-09-20), pages 12168-12172, XP055383704, ISSN: 2320-3765, DOI: 10.15662/ijareeie.2014.0309047
- Dr Hans-Gerd Brummel: "ON-LINE MONITORING OF POWER PLANTS", Siemens Power Generation (PG), 1 January 2006 (2006-01-01), pages 1-13, XP055383807, Retrieved from the Internet: URL:http://m.energy.siemens.com/mx/pool/hq /energy-topics/pdfs/en/gas-turbines-power- plants/1_Online_Monitoring_of_PP.pdf [retrieved on 2017-06-21]

## Description

### BACKGROUND OF THE INVENTION

The subject matter disclosed herein relates to power plants, particularly systems and methods for improving reporting and control of the power plant based on fuel quality of the power plant.

AU 2012 100 395 A4 describes a system and method for determining whether a transported fuel has been adulterated during the fuel's transport, e.g. whether expensive diesel has been replaced with cheaper petrol (gasoline), whether diesel has been removed from a road tanker, dishonestly used or sold, whether some of the fuel has been replaced by water. The system comprises a fuel data receiver arranged to receive data indicative of at least one measured property (like mass flow, density, volume, temperature) of the fuel at each of a plurality of positions on a route along which the fuel is transported. The system also comprises a fuel data comparator arranged to compare the fuel data from two of the plurality of positions when received and to determine a discrepancy between the fuel data relating to the two positons. If the compared fuel data falls outside a tolerated discrepancy range, the person who is responsible for transporting the fuel between the two positions on the route is determined and an alert or discrepancy signal is generated.

### BRIEF DESCRIPTION OF THE INVENTION

Certain embodiments commensurate in scope with the originally claimed invention are summarized below.

### SUMMARY OF THE INVENTION

According to the invention there is provided a plant-integrated measurement and monitoring system, comprising: a first sampling port disposed in a first set of pipelines in the power plant that convey liquid fuel from a liquid fuel source to a dirty storage tank, the first sampling port configured to provide a load sample of the fuel, the load sample representing the fuel provided by the fuel source prior to storage, processing, or both; a second sampling port disposed in a second set of pipelines in the power plant downstream from a downstream component, the downstream component configured to store, process, or store and process the fuel downstream from the dirty storage tank, and the second sampling port configured to provide a sample of the fuel downstream of the downstream component representative of fuel after processing, storage, or both of the fuel by the downstream component; and an automated analyzer device configured to: analyze the load sample to determine quality attributes of the load sample; analyze the post-downstream component sample to determine quality attributes of the post-downstream component; and provide the quality attributes of the load sample and the quality attributes of the post-downstream component to a distributed control system, enabling the distributed control system to identify anomalies in the fuel and attribute the anomalies as either an initial quality of the fuel as it is supplied by the fuel source or likely caused by the downstream component. The plant-integrated measurement and monitoring system is further configured to report the conditions of and/or adjust the operation of equipment in the power plant based in part on an analyzed quality of the liquid fuel at particular areas of the power plant.

The invention further provides a method, comprising: receiving, from a first sampling port in a power plant, a load sample of liquid fuel, the load sample representing the liquid fuel provided by a fuel source prior to storage, processing, or both; receiving, from a second sampling port in the power plant, a sample of the fuel downstream of a downstream component representative of fuel after processing, storage, or both of the fuel by the downstream component; analyzing, via an automated analyzer device, the load sample to determine quality attributes of the load sample; analyzing, via the automated analyzer device, the post-downstream component sample to determine quality attributes of the post-downstream component; and providing, from the automated analyzer device, the quality attributes of the load sample and the quality attributes of the post-downstream component to a distributed control system, enabling the distributed control system to: identify anomalies in the fuel; and attribute the anomalies as either an initial quality of the fuel as it is supplied by the fuel source or likely caused by the downstream component. The method further comprises reporting the conditions of and/or adjusting the operation of equipment in the power plant based in part on an analyzed quality of the liquid fuel at particular areas of the power plant

The invention also provides a tangible, non-transitory, machine-readable medium, comprising machine-readable instructions, in accordance with claim 17.

### BRIEF DESCRIPTION OF THE DRAWINGS

These and other features, aspects, and advantages of the present invention will become better understood when the following detailed description is read with reference to the accompanying drawings in which like characters represent like parts throughout the drawings, wherein:
FIG. 1 is a block diagram of an embodiment of a fuel-fed power plant with fuel analysis circuitry, in accordance with an embodiment;
FIG. 2 is a line chart, illustrating a relationship between contaminant concentration of fuel and lifetime of fuel-fed components of the power plant;
FIG. 3 is a flowchart illustrating a process for observing and analyzing power plant fuel used in the power plant of FIG. 1, in accordance with an embodiment;
FIG. 4 is a block diagram illustrating a device for analyzing fuel, in accordance with an embodiment;
FIG. 5 is a flowchart, illustrating a process for electronic notification and control of a power plant based upon analyzed fuel quality, in accordance with an embodiment;
FIG. 6 is a flowchart illustrating a process for controlling fuel loading based upon fuel quality, in accordance with an embodiment; and
FIG. 7 is a flowchart illustrating a process for controlling the power plant based upon fuel quality, in accordance with an embodiment.

### DETAILED DESCRIPTION OF THE INVENTION

One or more specific embodiments of the present invention will be described below. In an effort to provide a concise description of these embodiments, all features of an actual implementation may not be described in the specification. It should be appreciated that in the development of any such actual implementation, as in any engineering or design project, numerous implementation-specific decisions must be made to achieve the developers' specific goals, such as compliance with system-related and business-related constraints, which may vary from one implementation to another. Moreover, it should be appreciated that such a development effort might be complex and time consuming, but would nevertheless be a routine undertaking of design, fabrication, and manufacture for those of ordinary skill having the benefit of this disclosure.

When introducing elements of various embodiments of the present invention, the articles "a," "an," "the," and "said" are intended to mean that there are one or more of the elements. The terms "comprising," "including," and "having" are intended to be inclusive and mean that there may be additional elements other than the listed elements.

The embodiments disclosed herein relate to a system and method for improving efficiency of a power plant, by reporting on and/or adjusting the operation of equipment (e.g., a condenser, turbine, etc.) in the power plant based in part on a near-real-time analysis of fuel, water, and/or oil characteristics of the power plant.

Power plant systems have developed across the globe. Equipment of these power plant systems may rely on fuel, water, oil and other fluids to facilitate the plant operations. As may be appreciated, certain quality standards of these fluids may be relied upon for proper functioning of the equipment. For example, certain fuel particulate levels, contaminant levels, etc. specifications may be provided by an equipment vendor, detailing particular thresholds for the fluids that will help ensure proper functioning of the equipment.

Unfortunately, given the vast number of fuel sources, fuel delivery systems, plant maintenance, global standards, etc., the quality of these fluids may vary significantly from time to time. For example, liquid fuel in certain areas may include high particulate, may include water and/or sediments, contaminants, and/or jelly deposits, which may degrade the quality of fuel for the power plant.

Typically, plant operations have assumed that incoming fuel meets the threshold specifications provided by a vendor. The plants may include monitoring that determines whether the equipment is functioning properly. However, this may only determine a problem in a reactionary manner, as the problems arise, due to fuel or other fluids not meeting these specifications. This may result in the Balance of Plant (BOP) system becoming fowled (e.g., contaminated), which can ultimately lead to subsequent engine damage, if proper reactive measures are not taken.

Such reactive measures can be quite costly. For example, when a pressure drop is found, indicating that a filter is clogged, there may be significant troubleshooting needed to determine an actual cause of the clogging. Further, it may be costly, both with time and money to remedy the issue, as the piping, etc. may need flushing.

The embodiments provided herein provide systems and methods for proactively alerting and/or acting upon an analysis of plant fluids (e.g., fuel, water, oil, etc.). For example, data logs, digital twin applications (e.g., digital 3D modeling of the power plant), plant maintenance scheduling, etc. may be updated based upon plant fluid analysis.

As discussed below, the power plant may include equipment, such as a compressor, a combustor, a gas turbine engine, a steam cycle, etc. The sensors may include flow rate sensors, acoustical wave sensors, temperature sensors, pressure sensors, humidity sensors, composition sensors, or any combination thereof. The controller may also receive data output by other sensors that are configured to measure operating conditions of other fluids of the power plant system, such as the compressor, the gas turbine, or other components. As discussed in more detail below, in some embodiments, fuel samples, water samples, lube oil samples, etc. may be obtained from particular areas in the power plant. These samples may be analyzed to provide pro-active reporting and/or control. For example, these samples may be analyzed to measure particular characteristics of the fluids, such as color, particulates (e.g., size and distribution) and contaminant identification (e.g., an identified particular type of contaminant, such as from the following list of contaminates, for example, Na, K, Li, V, Mg, Pb, Ni, Ca Mn, Cr, Si, Fe, Al, Cu, Zn).

For example, the controller may use the data output by the sensor to adjust the power usage of the condenser, as the load of the power plant changes. In some embodiments, fuel may be re-directed for additional treatment, diversion, etc. Further, operation of one or more of the components of the power plant may be altered based upon the outputted sensor data. For example, component operation may be reduced when increased contaminants are present in fuel. For example, the speed of fans within each condenser may be adjusted, the pitch of the fan blades may be adjusted, etc.

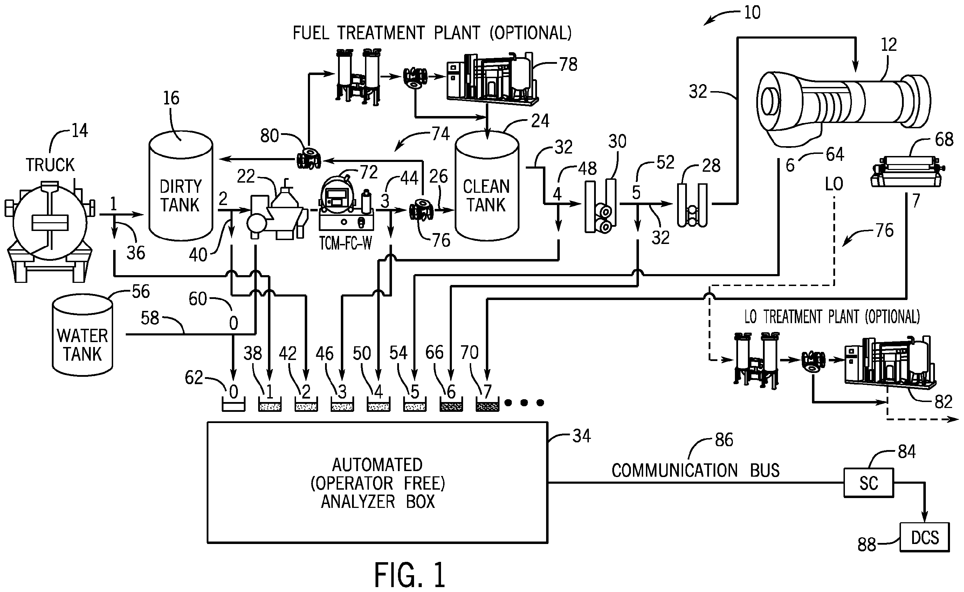

Turning now to the drawings, FIG. 1 is a block diagram of an embodiment of a power plant 10 having a gas turbine engine 12. The gas turbine engine 12 may be powered by fuel that is supplied by a fuel delivery system, such as the fuel truck 14. A dirty tank 16 may receive the fuel, via pipeline(s) 18. Further, the plant 10 may provide initial fuel treatment by supplying the fuel from pipeline(s) 20 to a centrifuge 22, where particulates and may be separated from the fuel. The treated fuel may be provided to a clean tank 24, via pipeline(s) 26 for storage until needed for use by the gas turbine engine 12. The fuel may be provided to the gas turbine engine 12 (e.g., after further downstream processing by filter 28 and/or other components 30), via pipeline(s) 32.

As will be discussed in more detail below, fluids of the plant 10 (e.g., the fuel, water, and lube oil) may be analyzed, at certain points of the power plant 10 operations, to determine certain characteristics (e.g., identify particular contaminates, particulate concentrations, etc.) of the fluids at these certain points. For example, in the embodiment of FIG. 1, an automated analyzer box 34 may receive component samples via one or more ports of the power plant 10. For example, when the plant 10 is equipped with supplementary filtering and/or conditioning equipment that can be engaged to correct fluid quality, additional sampling points may be provided to assess an effectiveness of these systems to help predict how long the plant 10 can operate before hardware distress may occur.

In one embodiment, Portl 36 may provide fuel samples from the pipeline(s) 18. Portl 36 may provide samples 38 of the initial fuel quality straight from the fuel supply source (e.g., the truck 14), prior to downstream processing and/or storage at the power plant 10. Accordingly, the automated analyzer box 34 may understand an initial fuel quality that is supplied to the power plant 10.

Additionally, Port2 40 may be provided at the pipeline(s) 20, supplying fuel from the dirty tank 16. Accordingly, these samples 42 may represent the state of the fuel after storage in the dirty tank 16. This may be useful in attributing fuel contamination to the dirty tank 16.

Port3 44 may be positioned after the centrifuge 22. The fuel sample 46 may provide an indication of the fuel quality after processing by the centrifuge 22, which may be useful to determine the effectiveness of the processing by the centrifuge 22.

Port4 48 may be placed after the clean tank 24. The fuel sample 50 may provide an indication of the fuel quality after storage in the clean tank 24. These fuel samples 50 may be useful to attribute contamination to the clean tank 24.

Port5 52 may be placed in the pipeline(s) 32 after additional equipment 30. The fuel samples 66 may be used to determine the fuel quality after the additional equipment 30 and/or before the filter 28.

As mentioned above, additional fluids may be analyzed. For example, the power plant may use water, which may be stored in the water tank 56. The pipeline(s) 58 may supply the water. PortO 60 of the gas turbine engine 12 may provide water samples 62 to the automated analyzer box 34. Further, lube oil samples may be provided to the automated analyzer box 34. For example, Port6 64 may provide samples 54 and additional equipment 68 that uses the lube oil may provide additional lube oil samples 70 to the automated analyzer box 34.

The automated analyzer box 34 and or additional sensors (e.g., the fuel quality sensor 72) may provide an indication of the quality of the fluids. When the quality is below a particular threshold branching pipeline(s), such as fuel treatment branching pipeline(s) 74 and/or lube oil treatment branching pipeline(s) 76 may divert the fluids for additional treatment. For example, when the fuel quality is below a threshold value, the valve 76 may be actuated to divert the fuel to the fuel treatment branching pipeline(s) 74 instead of storing the inadequate fuel in the clean tank 24.

The fuel treatment branching pipeline(s) 74 may divert the fuel to the fuel treatment plant 78 or send the fuel back to the dirty tank 16 for additional treatment by the centrifuge 22. The fuel quality sensor 72 and/or the automated analyzer box 34 may determine characteristics of the fuel and determine which option (e.g., fuel treatment plant 78 or additional centrifuge 22 processing). For example, small amounts of contamination may warrant additional centrifuge 22 treatment, while higher levels of contamination may warrant treatment at the fuel treatment plant 78. Accordingly, the valve 80 may be actuated accordingly, based upon the fuel quality analysis prior to the clean tank 24 (e.g., via samples 46).

Additionally, the automated analyzer box 34 may determine when the lube oil is below a threshold quality level. When below a threshold quality level, the lube oil may be diverted to a lube oil treatment plant 82 and/or alternative lube oil treatment equipment.

As will be discussed in more detail below, the automated analyzer box 34 may determine the containments and/or other characteristics of fluids of the power plant 10. The automated analyzer box 34 may be connected to a signal-conditioning device 84 via a communications bus 86. The signal conditioning device 84 may receive data indicative of the component quality and/or other characteristics via the communications bus 86. The signal-conditioning device 84 may convert this data into signals interpretable by a control system (e.g., distributed control system 88). Based upon the signals provided by the signal- conditioning device 84, the control system may provide alerts and/or control of equipment in the power plant 10.

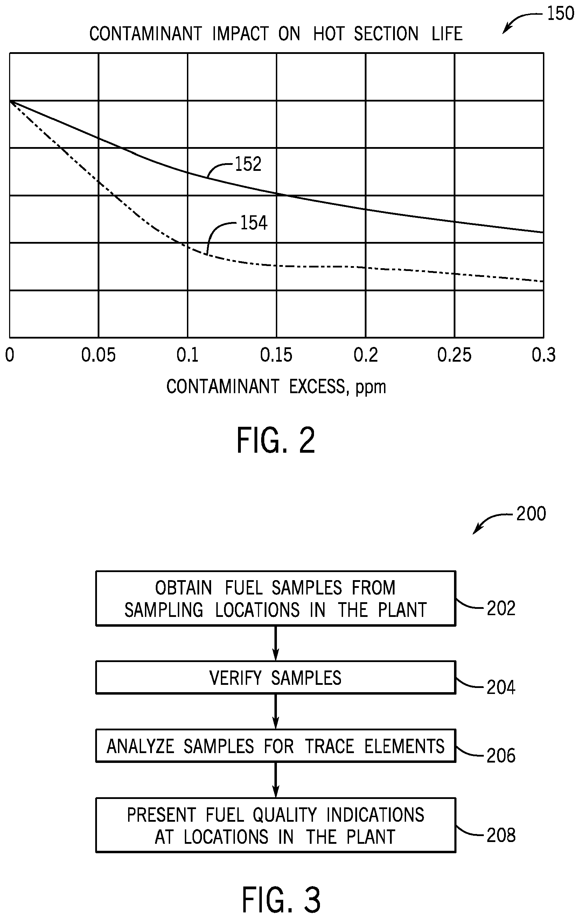

FIG. 2 is a line chart 150, illustrating a relationship between contaminant concentration of fuel and lifetime of fuel-fed components of the power plant 10. The -Axis provides an indication of a contaminant excess concentration in parts per million (ppm). The Y-Axis provides an indication of a life expectancy of the hot section (e.g., the combustor, turbine, afterburner, exhaust, etc.) of the gas turbine engine 12. The line 152 illustrates the effect of contaminant "A" and the line 154 illustrates the effect of contaminant "B". As illustrated by lines 152 and 154, as the contaminants increase, the life of the hot section equipment decreases. For example, at a 0 contaminant excess, the life of the hot section equipment is much higher than at a higher ppm content. Accordingly, as may be appreciated, the current techniques that analyze fluids throughout the power plant 10 may be useful in proactively notifying an operator and/or controlling operations in the plant 10, based upon contaminant levels.

FIG. 3 is a flowchart illustrating a process 200 for observing and analyzing power plant fuel used in the power plant of FIG. 1, in accordance with an embodiment. The process 200 begins by obtaining fuel samples (and/or other component samples) from Sampling locations in the plant 10 (block 202). For example, as mentioned above with regards to FIG. 1, samples may be provided to the automated analyzer box 34 from the ports (e.g., Porto 60, Portl 36, Port2 40, Port3 44, Port4 48, Port5 52, Port6 64, Port7 68).

Next, the samples may be verified (block 204). For example, optical techniques may be calibrated to measure liquid fuel opacity and/or color and/or may detect water content and/or particular loading. The system may further include automatic online particle sampling and binning devices.

The component samples may then be analyzed for trace elements (block 206). The samples are drawn from a pipe system designed to provide a continuous flow of fresh fluid at the analyzer location. As will be discussed in more detail below, the analyzer box 34, in one embodiment, is a robot (e.g., using rotating disk electrode atomic emission spectrometry) that receives the samples, executes analysis and provides a digitized signal encoding of the results of the analyses.

After analysis, present fuel quality indications at the various sampling locations in the plant 10 may be supplied for downstream altering and/or control (block 208). For example, a signal-conditioning device 84 may monitor for digital signals from the analyzer box 34. The signal-conditioning device 84 may sequence and condition the signals received from analyzer box 34 to provide control system discernable data to the distributed control system 88.

The process 200 may be implemented on a periodic basis. For example, the process 200 may be completed in near-real time, resulting in near-real time alerts and/or control. For example, in certain embodiments, the process 200 may be completed approximately every 5 minutes during power plant 10 operation.

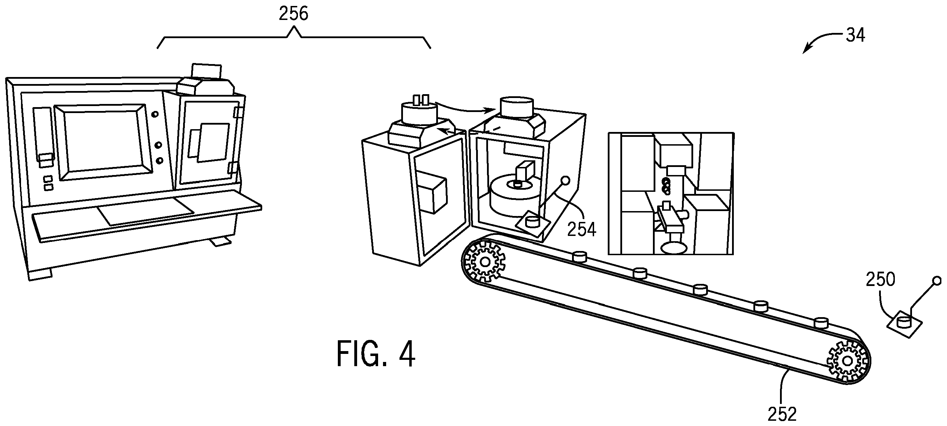

Turning now to a discussion of the automated analyzer box, FIG. 4 is a block diagram illustrating an embodiment of an automated analyzer device 34 for analyzing fuel, in accordance with an embodiment. As mentioned above with regard to FIG. 1, samples 250 may be prepared. The samples 250 may be positioned on a conveyor system 252. In some embodiments, some samples may be empty or contain a neutral liquid or a calibration standard to facilitate operation of the analyzer.

A robotic arm 254 may transfer the samples 250 (e.g., one at a time) to the analyzer 256. As mentioned above, the analyzer 256 may use rotating disk electrode technology to identify contaminate and/or particulate concentration levels. The analysis results may be provided from the analyzer 256 to a downstream component, such as a distributed control system 88.

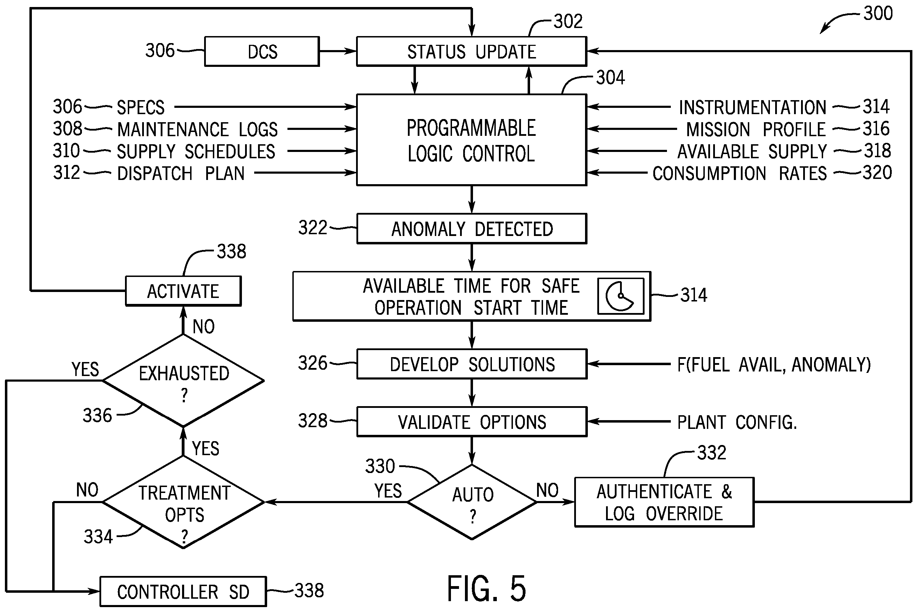

FIG. 5 is a flowchart, illustrating a process 300 for electronic notification and/or control of a power plant 10 based upon analyzed fuel, water, and/or lube oil quality, in accordance with an embodiment. As discussed with regard to FIG. 1, the distributed control system 88 may provide status updates 302 to a programmable logic controller (PLC) 304. The status updates 302 include an analysis of fluid samples taken from certain points in the power plant 10.

The PLC 304 may receive these status updates 302, along with other plant 10 information, such as equipment specifications 306, maintenance logs 308, supply schedules 310, dispatch plan 312, instrumentation 314, mission profile 316, available supply 318, consumption rates 320, etc. Based upon this received data, the PLC 304 may detect anomalies (e.g., non-conformity of the liquids as opposed to the plant requirements defined in the specifications 306) (block 322).

After detecting an anomaly, the PLC 304 may determine an amount of time remaining for safe operation, in light of the anomaly, and start a timer counting down an amount of time before operation of the plant 10 is to be altered (block 324). For example, relatively highly contaminated fuel may reduce a safe operation time for a gas turbine engine 12. Accordingly, the PLC 304 may determine a relatively low safe operation time. Additionally, the PLC 304 may trigger notifications (e.g., alarms, etc.) based upon the severity of the detected anomaly.

The PLC 304 (or other circuitry) may develop solutions for the anomaly based upon the available fuel supply 318, and the determined anomaly (block 326). For example, a Balance of Plant (BOP) capability and risk analysis (e.g., based upon the maintenance logs 308, instrumentation 314, specifications 306, mission profile 316, etc.) may be used to determine if the anomaly (e.g., the particular level of insufficient fuel quality) may be accepted and in what amount, such that plant 10 operations may continue. Supply vs. Demand, market conditions, and risk-based analyses can be implemented to maximize profit and/or minimize costs. Decision trees may take into account available redundant or optional filtration and/or conditioning systems to maximize run time and minimize impact on the equipment.

In some embodiments, a more complex analysis may detect if the identified anomaly is a direct result of low-quality fuel delivered to the plant 10 or due to malfunction in a particular portion of the plant BOP. For example, because the sampling locations are tracked with the samples, samples that indicate low-quality fuel can be attributed to particular portions of the plant 10. Plant instrumentation 314 and engine mission profile 316 may be integrated into the analysis to derive a comprehensive view of plant 10 health.

The PLC 304 (or other circuitry) may validate the options (block 328) to determine their viability with the current conditions. For example, detailed records including fuel, water, and lube oil condition along with operation history are used to enable Condition Based Maintenance. These records may establish remaining life of the components of the power plant 10 and risks involved in continued operations with the contaminated liquids. The potential solutions are ranked based upon their risk, plant configuration, and generation plans.

The PLC 304 (or other circuitry) may determine whether the control system of the power plant 10 is set to implement solution options automatically (decision block 330). If automatic implementation is not set, the plan may only be implemented after a user is authenticated and an override of current operations is selected by the user (block 332). Otherwise, if automatic implementation is set, the PLC 304 (or other circuitry) determines if treatment options for the anomaly are available (decision block 334). If there are treatment options, the PLC 304 (or other circuitry) determines whether the treatment options are exhausted (decision block 336). If there are not treatment options or the treatment options are exhausted, a controlled shutdown is performed by the end of the timer started in block 324 (block 338). However, when treatment options exist and have not been exhausted, the best of the available options (as determined during the validation option in block 328) is activated (block 338). Once these changes are implemented, the process 300 restarts, determining if the changes have enhanced the plant 10 operations and determining new safe operation times, etc.

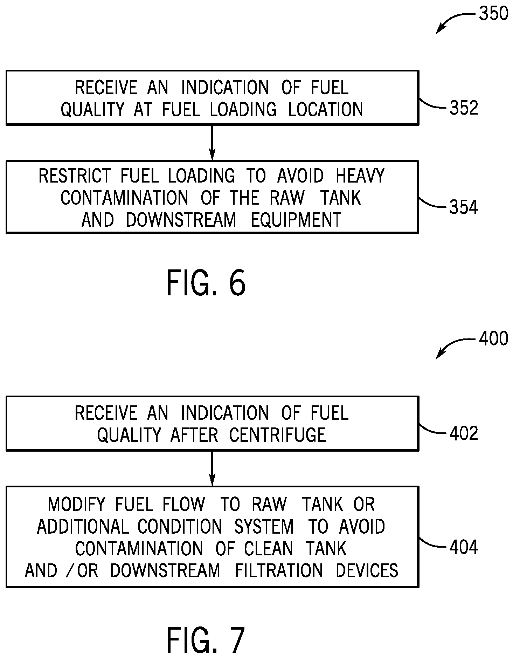

As mentioned above, sometimes the initial fuel supply does not meet minimum requirements. FIG. 6 is a flowchart illustrating a process 350 for controlling fuel loading based upon an initial fuel quality, in accordance with an embodiment. First, an indication of fuel quality at the fuel loading location is received (block 352). For example, returning to FIG. 1, samples from the fuel analysis of Portl 36 may provide an indication of poor initial fuel quality. Based upon this information, the fuel loading maybe restricted to avoid heavy contamination of the raw tank and downstream equipment. For example, valves may be actuated to cut access to the dirty tank 16. Additionally and/or alternatively, an alert of the poor fuel load may be provided via a human machine interface (HMI), enabling a power plant 10 operator to stop the fuel load manually.

FIG. 7 is a flowchart illustrating a process 400 for controlling the power plant 10 based upon fuel quality, in accordance with an embodiment. First, an indication of the fuel quality after the centrifuge (e.g., centrifuge 22 of FIG. 1) (block 402). The fuel flow to the clean tank 24 may be removed and/or additional conditioning of the fuel may be implemented to avoid contamination of the clean tank and/or downstream filtration devices (block 404). For example, as mentioned above, regarding FIG. 1, the valve 76 may redirect fuel to valve 80, which may either direct the fuel to the fuel treatment plant 78 and/or back to the dirty tank 16, such that the fuel undergoes centrifuge 22 treatment again.

Various instructions, methods, and techniques described herein may be considered in the general context of computer-executable instructions, such as program modules, executed by one or more computers or other devices. Generally, program modules include routines, programs, objects, components, data structures, etc., for performing particular tasks or implementing particular abstract data types. These program modules and the like may be executed as native code or may be downloaded and executed, such as in a virtual machine or other just-in-time compilation execution environment. The functionality of the program modules may be combined or distributed as desired in various embodiments. An implementation of these modules and techniques maybe stored on some form of computer-readable storage media.

Technical effects of the invention include a system and method for improving efficiency of a power plant, based in part on reporting the conditions of and/or adjusting the operation of equipment in the power plant based in part on an analyzed quality of the fuel at particular areas of the power plant. A controller uses the data output by fuel, water, and/or, oil analysis sensors to provide alerts and actions regarding the operation of the power plant. By providing alerts and/or actions based upon fuel, water, and/or oil analysis, pro-active actions may be performed, resulting in prolonged life-expectancy of the power plant equipment, a reduction in power-plant outages, etc.

Technical effects of the current system and methods include enabling condition based maintenance by providing advanced analytics to interpret current operational fluid qualities. Further, the current techniques provide mitigation plans, taking into account plant configuration and operation history and/or a risk/reward analysis. Accordingly, despite variability in quality of supplied liquid fuel and/or inadequate plant maintenance and/or inadequate operation of plant conditioning systems that cause varied fluid qualities, reliable operation and control of the plant 10 may be maintained, resulting in increased operational efficiencies with decreased downtime.

This written description uses examples to disclose the invention, including the best mode, and also to enable any person skilled in the art to practice the invention, including making and using any devices or systems and performing any incorporated methods. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal language of the claims.

## Claims

1. A power plant-integrated measurement and monitoring system, comprising:
a first sampling port (36) disposed in a first set of pipelines in the power plant (10) that convey liquid fuel from a liquid fuel source to a dirty storage tank (16), the first sampling port configured to provide a load sample (38) of the fuel, the load sample representing the fuel provided by the fuel source prior to storage, processing, or both;
a second sampling port (44) disposed in a second set of pipelines in the power plant (10) downstream from a downstream component (22), the downstream component configured to store, process, or store and process the fuel downstream from the dirty storage tank, and the second sampling port configured to provide a sample (46) of the fuel downstream of the downstream component representative of fuel after processing, storage, or both of the fuel by the downstream component;
and an automated analyzer device (34) configured to:
analyze the load sample (38) to determine quality attributes of the load sample;
analyze the post-downstream component sample (46) to determine quality attributes of the post-downstream component (22); and
provide the quality attributes of the load sample (38) md the quality attributes of the post-downstream component sample (46) to a distributed control system (88) (DCS), the distributed control system configured to identify anomalies in the fuel and attribute the anomalies as either an initial quality of the liquid fuel as it is supplied by the fuel source or likely caused by the downstream component;
wherein the plant-integrated measurement and monitoring system is configured to report the conditions of and/or adjust the operation of equipment in the power plant (10) based in part on an analyzed quality of the liquid fuel samples provided at said first and second sampling ports (36, 44) of the power plant (10).

2. The plant-integrated measurement and monitoring system of claim 1, wherein the downstream component (22) comprises a centrifuge.

3. The plant-integrated measurement and monitoring system of claim 2, comprising:
a third sampling port (40) disposed between the dirty storage tank (16) and the centrifuge (22), the third sampling port configured to provide a post-dirty storage tank sample (42) of the fuel representative of fuel after storage in the dirty fuel tank.

4. The plant-integrated measurement and monitoring system of claim 3, wherein the automated analyzer device (34) is configured to analyze the post-dirty storage tank sample (42) to determine quality attributes of the post-dirty storage tank sample;
provide the quality attributes of the post-dirty storage tank sample to the distributed control system, enabling the distributed control system to determine if the identified anomalies should be attributed to storage in the dirty storage tank.

5. The plant-integrated measurement and monitoring system of claim 1, comprising a first lube oil sampling port (64) configured to provide a first lube oil sample (54) to the automated analyzer device, wherein the automated analyzer device is configured to:
analyze the first lube oil sample (54) to determine quality attributes of the first lube oil sample; and
provide the quality attributes of the first lube oil sample to the distributed control system (88), enabling the distributed control system to determine if the identified anomalies are likely a result of lube oil.

6. The plant-integrated measurement and monitoring system of claim 5, wherein the first lube oil sampling port (64) is disposed at a gas turbine engine (12).

7. The plant-integrated measurement and monitoring system of claim 5, comprising a first water sampling port (60) configured to provide a first water sample (62) to the automated analyzer device, wherein the automated analyzer device is configured to:
analyze the first water sample (62) to determine quality attributes of the first water sample; and
provide the quality attributes of the first water sample to the distributed control system, enabling the distributed control system to determine if the identified anomalies are likely a result of water.

8. The plant-integrated measurement and monitoring system of claim 1, wherein the quality attributes of the load sample (38), the quality attributes of the post- downstream component sample (46), or both comprise: a particulate concentration, a water concentration, identification of contaminants, or any combination thereof.

9. The plant-integrated measurement and monitoring system of claim 1, wherein the automated analyzer device comprises:
a conveyer system (252) configured to provide samples (250) from a sample preparation site to a liquid analyzer; and
the liquid analyzer is configured to determine quality attributes of the samples using a rotating disk electrode.

10. The plant-integrated measurement and monitoring system of claim 9, wherein the automated analyzer device (34) is configured to identify contaminants in samples when they exist, the contaminates comprising: Sodium (Na), Potassium (K), Lithium (Li), Vanadium (V), Magnesium (Mg), Lead (Pb), Nickel (Ni), Calcium (Ca), Manganese (Mn), Chromium (Cr), Silicon (Si), Iron (Fe), Aluminum (Al), Copper (Cu), and Zinc (Zn).

11. The plant-integrated measurement and monitoring system of claim 9, comprising:
a signal-conditioning device (84) configured to convert input signals into output signals interpretable by the distributed control system;
wherein the automated analyzer device is configured to provide the quality attributes as the input signals to the signal-conditioning device via a communications bus (86).

12. A power plant integrated measurement and monitoring method, comprising:
receiving, from a first sampling port (36) disposed in a first set of pipelines in a power plant (10) that convey liquid fuel from a liquid fuel source to a dirty storage tank (16), a load sample of liquid fuel, the load sample (38) representing the liquid fuel provided by the fuel source prior to storage, processing, or both;
receiving, from a second sampling port (44) disposed in a second set of pipelines in the power plant (10), a sample (46) of the fuel downstream from a downstream component representative of fuel after processing, storage, or both of the fuel by the downstream component (22);
analyzing, via an automated analyzer device (34), the load sample (38) to determine quality attributes of the load sample;
analyzing, via the automated analyzer device, the post-downstream component sample (46) to determine quality attributes of the post-downstream component; and
providing, from the automated analyzer device, the quality attributes of the load sample (38) and the quality attributes of the post-downstream component (22) sample (46) to a distributed control system (88), enabling the distributed control system to:
identify anomalies in the fuel; and
attribute the anomalies as either an initial quality of the fuel as it is supplied by the fuel source or likely caused by the downstream component;
wherein the method further comprises reporting the conditions of and/or adjust the operation of equipment in the power plant (10) based in part on an analyzed quality of the liquid fuel samples provided at said first and second sampling ports (36, 44) of the power plant (10).

13. The method of claim 13, wherein analyzing the load sample (38) to determine the quality attributes of the load sample comprises detecting a particulate concentration, a water concentration, identifying contaminants, or any combination thereof of the load sample.

14. The method of claim 13, wherein analyzing the sample (46) of the fuel downstream of the downstream component to determine the quality attributes of said sample comprises detecting a particulate concentration, a water concentration, identifying contaminants, or any combination thereof of said sample.

15. The method of claim 13, wherein the providing of the quality attributes of the load sample (38) and the quality attributes of the post-downstream component (22) to the distributed control system, system (DCS), comprises:
providing quality and location data regarding the fuel from the automated analyzer device (34) via a communication bus (86) to signal-conditioning device (84);
converting the quality and location data to a DCS signal interpretable by the distributed control system; and
providing the DCS signal to the distributed control system (88).

16. The method of claim 13, comprising:
receiving at least one water sample (62) and at least one lube oil sample (54) from respective water ports (60) and lube oil ports (64);
analyzing the at least one water sample and the at least one oil sample to determine quality attributes of the at least one water sample and the at least one oil sample; and
providing the quality attributes of the at least one water sample and the at least one oil sample to the distributed control system, enabling the distributed control system to:
identify anomalies in power plant water, power plant lube oil, or both; and
identify an associated location based upon a location where the at least one water sample and the at least one oil sample were sourced.

17. A tangible, non-transitory, machine-readable medium, comprising machine-readable instructions, configured to cause the system of claim 1 to execute the steps of the method of claim 12.

## Patentansprüche

1. Kraftwerksintegriertes Mess- und Überwachungssystem, umfassend:
einen ersten Probenahmeanschluss (36), der in einem ersten Satz von Pipelines in dem Kraftwerk (10) angeordnet ist, die Flüssigbrennstoff von einer Flüssigbrennstoffquelle zu einem Schmutzlagertank (16) befördern, wobei der erste Probenahmeanschluss konfiguriert ist, um eine Lastprobe (38) des Brennstoffs bereitzustellen, wobei die Lastprobe den Brennstoff darstellt, der von der Brennstoffquelle vor der Lagerung, Verarbeitung oder beidem bereitgestellt wird;
einen zweiten Probenahmeanschluss (44), der in einem zweiten Satz von Pipelines in dem Kraftwerk (10) stromabwärts von einer stromabwärtigen Komponente (22) angeordnet ist, wobei die stromabwärtige Komponente konfiguriert ist, um den Brennstoff stromabwärts von dem Schmutzlagertank zu speichern, zu verarbeiten oder zu speichern und zu verarbeiten, und der zweite Probenahmeanschluss konfiguriert ist, um eine Probe (46) des Brennstoffs stromabwärts der stromabwärtigen Komponente bereitzustellen, die für Brennstoff nach dem Verarbeiten, Speichern oder beidem des Brennstoffs durch die stromabwärtige Komponente repräsentativ ist;
und eine automatisierte Analysevorrichtung (34), die konfiguriert ist zum:
Analysieren der Lastprobe (38), um Qualitätsattribute der Lastprobe zu bestimmen;
Analysieren der Probe der post-stromabwärtigen Komponente (46), um Qualitätsattribute der post-stromabwärtigen Komponente (22) zu bestimmen; und
Bereitstellen der Qualitätsattribute der Lastprobe (38) und der Qualitätsattribute der post-stromabwärtigen Komponentenprobe (46) einem verteilten Steuersystem (88) (DCS), wobei das verteilte Steuersystem so konfiguriert ist, dass es Anomalien im Brennstoff identifiziert und die Anomalien entweder als eine anfängliche Qualität des Flüssigbrennstoffs, wie er von der Brennstoffquelle geliefert wird, oder wahrscheinlich durch die stromabwärtige Komponente verursacht, zuweist;
wobei das kraftwerksintegrierte Mess-und Überwachungssystem konfiguriert ist, um die Bedingungen von und/oder den Betrieb von Ausrüstung im Kraftwerk (10) basierend teilweise auf einer analysierten Qualität der Flüssigbrennstoffproben, die an dem ersten und dem zweiten Probenahmeanschluss (36, 44) des Kraftwerks (10) bereitgestellt werden, zu melden.

2. Kraftwerksintegriertes Mess- und Überwachungssystem nach Anspruch 1, wobei die stromabwärtige Komponente (22) eine Zentrifuge umfasst.

3. Kraftwerksintegriertes Mess- und Überwachungssystem nach Anspruch 2, umfassend:
einen dritten Probenahmeanschluss (40), der zwischen dem Schmutzlagertank (16) und der Zentrifuge (22) angeordnet ist, wobei der dritte Probenahmeanschluss konfiguriert ist, um eine Post-Schmutzlagertankprobe (42) des Brennstoffs bereitzustellen, die für Brennstoff nach Lagerung im Schmutzbrennstofftank repräsentativ ist.

4. Kraftwerksintegriertes Mess- und Überwachungssystem nach Anspruch 3, wobei die automatisierte Analysevorrichtung (34) konfiguriert ist, um die Post-Schmutzlagertankprobe (42) zu analysieren, um Qualitätsattribute der Post-Schmutzlagertankprobe zu bestimmen;
Bereitstellen der Qualitätsattribute der Post-Schmutzlagertankprobe dem verteilten Steuersystem, wodurch dem verteilten Steuersystem ermöglicht versetzt wird, zu bestimmen, ob die identifizierten Anomalien der Lagerung in dem Schmutzlagertank zuzuschreiben sind.

5. Kraftwerksintegriertes Mess- und Überwachungssystem nach Anspruch 1, umfassend einen ersten Schmierölprobenahmeanschluss (64), der konfiguriert ist, um der automatisierten Analysevorrichtung eine erste Schmierölprobe (54) bereitzustellen, wobei die automatisierte Analysevorrichtung konfiguriert ist zum:
Analysieren der ersten Schmierölprobe (54), um Qualitätsattribute der ersten Schmierölprobe zu bestimmen; und
Bereitstellen der Qualitätsattribute der ersten Schmierölprobe dem verteilten Steuersystem (88), wodurch es dem verteilten Steuersystem ermöglicht wird, zu bestimmen, ob die identifizierten Anomalien wahrscheinlich Schmieröl zuzuschreiben sind.

6. Kraftwerksintegriertes Mess- und Überwachungssystem nach Anspruch 5, wobei der erste Schmierölprobenahmeanschluss (64) an einem Gasturbinentriebwerk (12) angeordnet ist.

7. Kraftwerksintegriertes Mess- und Überwachungssystem nach Anspruch 5, umfassend einen ersten Wasserprobenahmeanschluss (60), der konfiguriert ist, um der automatisierten Analysevorrichtung eine erste Wasserprobe (62) bereitzustellen, wobei die automatisierte Analysevorrichtung konfiguriert ist zum:
Analysieren der ersten Wasserprobe (62), um Qualitätsattribute der ersten Wasserprobe zu bestimmen; und
Bereitstellen der Qualitätsattribute der ersten Wasserprobe dem verteilten Steuersystem, wodurch es dem verteilten Steuersystem ermöglicht wird, zu bestimmen, ob die identifizierten Anomalien wahrscheinlich Wasser zuzuschreiben sind.

8. Kraftwerksintegriertes Mess- und Überwachungssystem nach Anspruch 1, wobei die Qualitätsattribute der Lastprobe (38), die Qualitätsattribute der post-stromabwärtigen Komponentenprobe (46) oder beide Folgendes umfassen: eine Partikelkonzentration, eine Wasserkonzentration, Identifizierung von Verunreinigungen oder eine beliebige Kombination davon.

9. Kraftwerksintegriertes Mess- und Überwachungssystem nach Anspruch 1, wobei die automatische Analysevorrichtung Folgendes umfasst:
ein Fördersystem (252), das konfiguriert ist, um Proben (250) von einer Probenvorbereitungsstelle an einen Flüssigkeitsanalysator bereitzustellen; und
der Flüssigkeitsanalysator konfiguriert ist, um Qualitätsattribute der Proben unter Verwendung einer rotierenden Scheibenelektrode zu bestimmen.

10. Kraftwerksintegriertes Mess- und Überwachungssystem nach Anspruch 9, wobei die automatisierte Analysevorrichtung (34) konfiguriert ist, Verunreinigungen in Proben zu identifizieren, wenn sie existieren, wobei die Verunreinigungen Folgendes umfassen: Natrium (Na), Kalium (K), Lithium (Li), Vanadium (V), Magnesium (Mg), Blei (Pb), Nickel (Ni), Calcium (Ca), Mangan (Mn), Chrom (Cr), Silicium (Si), Eisen (Fe), Aluminium (Al), Kupfer (Cu) und Zink (Zn).

11. Kraftwerksintegriertes Mess- und Überwachungssystem nach Anspruch 9, umfassend:
eine Signalaufbereitungsvorrichtung (84), die konfiguriert ist, um Eingangssignale in Ausgangssignale umzuwandeln, die von dem verteilten Steuersystem interpretiert werden können;
wobei die automatisierte Analysevorrichtung konfiguriert ist, um die Qualitätsattribute als die Eingangssignale an die Signalaufbereitungsvorrichtung über einen Kommunikationsbus (86) bereitzustellen.

12. Kraftwerksintegriertes Mess- und Überwachungsverfahren, umfassend:
Empfangen, von einem ersten Probenahmeanschluss (36), der in einem ersten Satz von Pipelines in einem Kraftwerk (10) angeordnet ist, die Flüssigbrennstoff von einer Flüssigbrennstoffquelle zu einem Schmutzlagertank (16) befördern, einer Lastprobe von Flüssigbrennstoff, wobei die Lastprobe (38) den Flüssigbrennstoff darstellt, der bereitgestellt wird durch; die Brennstoffquelle vor der Lagerung, Verarbeitung oder beidem;
Empfangen, von einem zweiten Probenahmeanschluss (44), der in einem zweiten Satz von Pipelines in dem Kraftwerk (10) angeordnet ist, einer Probe (46) des Brennstoffs stromabwärts von einer stromabwärtigen Komponente, die repräsentativ ist für Brennstoff nach der Verarbeitung, Speicherung oder beidem des Brennstoffs durch die stromabwärtige Komponente (22);
Analysieren, über eine automatisierte Analysevorrichtung (34), der Lastprobe (38) um Qualitätsattribute der Lastprobe zu bestimmen;
Analysieren, über die automatisierte Analysevorrichtung, der post-stromabwärtigen Komponentenprobe (46) über die automatisierte Analysevorrichtung, um Qualitätsattribute der post-stromabwärtigen Komponente zu bestimmen; und
Bereitstellen, von der automatisierten Analysevorrichtung, der Qualitätsattribute des Lastprobe (38) und der Qualitätsattribute der post-stromabwärtigen Komponenten- (22) Probe (46) an ein verteiltes Steuersystem (88), wodurch dem verteilten Steuersystem Folgendes ermöglicht wird:
Identifizieren von Anomalien in dem Brennstoff; und
Zuweisen der Anomalien als entweder eine anfängliche Qualität des Brennstoffs, wenn er von der Brennstoffquelle zugeführt wird oder wahrscheinlich durch die stromabwärtige Komponente verursacht wird;
wobei das Verfahren ferner das Berichten der Bedingungen der Ausrüstung in dem Kraftwerk (10) und/oder das Einstellen des Betriebs dieser teilweise basierend auf einer analysierten Qualität der Flüssigbrennstoffproben umfasst, die an den ersten und zweiten Probenahmeanschlüssen (36, 44) des Kraftwerks (10) bereitgestellt werden.

13. Verfahren nach Anspruch 13, wobei das Analysieren der Lastprobe (38), um die Qualitätsattribute der Lastprobe zu bestimmen, das Detektieren einer Partikelkonzentration, einer Wasserkonzentration, das Identifizieren von Verunreinigungen oder einer beliebigen Kombination davon der Lastprobe umfasst.

14. Verfahren nach Anspruch 13, wobei das Analysieren der Probe (46) des Brennstoffs stromabwärts der stromabwärtigen Komponente, um die Qualitätsattribute der Probe zu bestimmen, das Detektieren einer Partikelkonzentration, einer Wasserkonzentration, das Identifizieren von Verunreinigungen oder einer beliebigen Kombination davon der Probe umfasst.

15. Verfahren nach Anspruch 13, wobei das Bereitstellen der Qualitätsattribute der Lastprobe (38) und der Qualitätsattribute der post-stromabwärtigen Komponente (22) dem verteilten Steuersystem (DCS) umfasst:
Bereitstellen von Qualitäts- und Ortsdaten bezüglich des Brennstoffs von der automatisierten Analysevorrichtung (34) über einen Kommunikationsbus (86) an die Signalaufbereitungsvorrichtung (84);
Umwandeln der Qualitäts- und Standortdaten in ein DCS-SIGNAL, das durch das verteilte Steuersystem interpretierbar ist; und
Bereitstellen des DCS-SIGNALS an das verteilte Steuersystem (88).

16. Verfahren nach Anspruch 13, umfassend:
Empfangen mindestens einer Wasserprobe (62) und mindestens einer Schmierölprobe (54) von entsprechenden Wasseranschlüssen (60) und Schmierölanschlüssen (64);
Analysieren der mindestens einen Wasserprobe und der mindestens einen Ölprobe, um Qualitätsattribute der mindestens einen Wasserprobe und der mindestens einen Ölprobe zu bestimmen; und
Bereitstellen der Qualitätsattribute der mindestens einen Wasserprobe und der mindestens einen Ölprobe dem verteilten Steuersystem, wodurch dem verteilten Steuersystem Folgendes ermöglicht wird:
Identifizieren von Anomalien in Kraftwerkswasser, Kraftwerksschmieröl oder beidem; und
Identifizieren eines assoziierten Orts basierend auf einem Ort, an dem die mindestens eine Wasserprobe und die mindestens eine Ölprobe entnommen wurden.

17. Materielles, nichtflüchtiges, maschinenlesbares Medium, das maschinenlesbare Anweisungen umfasst, die konfiguriert sind, das System nach Anspruch 1 zu veranlassen, die Schritte des Verfahrens nach Anspruch 12 auszuführen.

## Revendications

1. Système de mesure et de surveillance intégré à une centrale électrique, comprenant :
un premier orifice d'échantillonnage (36) disposé dans un premier ensemble de pipelines dans la centrale électrique (10) qui transportent du carburant liquide depuis une source de carburant liquide vers un réservoir de stockage sale (16), le premier orifice d'échantillonnage configuré pour fournir un échantillon de charge (38) du carburant, l'échantillon de charge représentant le carburant fourni par la source de carburant avant le stockage, le traitement, ou les deux ;
un deuxième orifice d'échantillonnage (44) disposé dans un second ensemble de pipelines dans la centrale électrique (10) en aval d'un composant en aval (22), le composant en aval configuré pour stocker, traiter, ou stocker et traiter le carburant en aval depuis le réservoir de stockage sale, et le deuxième orifice d'échantillonnage configuré pour fournir un échantillon (46) du carburant en aval du composant en aval représentatif du carburant après le traitement, le stockage, ou les deux du carburant par le composant en aval ;
et un dispositif analyseur automatisé (34) configuré pour :
analyser l'échantillon de charge (38) pour déterminer des attributs de qualité de l'échantillon de charge ;
analyser l'échantillon (46) de composant post-aval pour déterminer des attributs de qualité du composant post-aval (22) ; et
fournir les attributs de qualité de l'échantillon de charge (38) et les attributs de qualité de l'échantillon (46) de composant post-aval vers un système de contrôle distribué (88) (DCS), le système de contrôle distribué configuré pour identifier des anomalies dans le carburant et attribuer les anomalies comme étant une qualité initiale du carburant liquide lorsqu'il est fourni par la source de carburant ou susceptible d'être provoqué par le composant en aval ;
dans lequel le système de mesure et de surveillance intégré à une centrale est configuré pour signaler les conditions de et/ou ajuster le fonctionnement de l'équipement dans la centrale électrique (10) en fonction en partie d'une qualité analysée des échantillons de carburant liquide fournis au niveau desdits premier et deuxième orifices d'échantillonnage (36, 44) de la centrale électrique (10).

2. Système de mesure et de surveillance intégré à une centrale selon la revendication 1, dans lequel le composant en aval (22) comprend une centrifugeuse.

3. Système de mesure et de surveillance intégré à une centrale selon la revendication 2, comprenant :
un troisième orifice d'échantillonnage (40) disposé entre le réservoir de stockage sale (16) et la centrifugeuse (22), le troisième orifice d'échantillonnage configuré pour fournir un échantillon de réservoir de stockage post-sale (42) du carburant représentatif du carburant après stockage dans le réservoir de carburant sale.

4. Système de mesure et de surveillance intégré à une centrale selon la revendication 3, dans lequel le dispositif analyseur automatisé (34) est configuré pour analyser l'échantillon de réservoir de stockage post-sale (42) pour déterminer des attributs de qualité de l'échantillon de réservoir de stockage post-sale ;
fournir les attributs de qualité de l'échantillon de réservoir de stockage post-sale au système de contrôle distribué, en permettant au système de contrôle distribué de déterminer si les anomalies identifiées doivent être attribuées au stockage dans le réservoir de stockage sale.

5. Système de mesure et de surveillance intégré à une centrale selon la revendication 1, comprenant un premier orifice d'échantillonnage d'huile lubrifiante (64) configuré pour fournir un premier échantillon d'huile lubrifiante (54) au dispositif analyseur automatisé, dans lequel le dispositif analyseur automatisé est configuré pour :
analyser le premier échantillon d'huile lubrifiante (54) pour déterminer les attributs de qualité du premier échantillon d'huile lubrifiante ; et
fournir les attributs de qualité du premier échantillon d'huile lubrifiante au système de contrôle distribué (88), permettant au système de contrôle distribué de déterminer si les anomalies identifiées sont susceptibles de résulter d'une huile lubrifiante.

6. Système de mesure et de surveillance intégré à une centrale selon la revendication 5, dans lequel le premier orifice d'échantillonnage d'huile lubrifiante (64) est disposé au niveau d'un moteur de turbine à gaz (12).

7. Système de mesure et de surveillance intégré à une centrale selon la revendication 5, comprenant un premier orifice d'échantillonnage d'eau (60) configuré pour fournir un premier échantillon d'eau (62) au dispositif analyseur automatisé, dans lequel le dispositif analyseur automatisé est configuré pour :
analyser le premier échantillon d'eau (62) pour déterminer les attributs de qualité du premier échantillon d'eau ; et
fournir les attributs de qualité du premier échantillon d'eau au système de contrôle distribué, permettant au système de contrôle distribué de déterminer si les anomalies identifiées sont susceptibles de résulter de l'eau.

8. Système de mesure et de surveillance intégré à une centrale selon la revendication 1, dans lequel les attributs de qualité de l'échantillon de charge (38), les attributs de qualité de l'échantillon (46) de composant post-aval, ou les deux comprennent : une concentration particulaire, une concentration en eau, une identification de contaminants, ou toute combinaison de ceux-ci.

9. Système de mesure et de surveillance intégré à une centrale selon la revendication 1, dans lequel le dispositif analyseur automatisé comprend :
un système de transporteur (252) configuré pour fournir des échantillons (250) d'un site de préparation d'échantillon à un analyseur de liquide ; et
l'analyseur de liquide est configuré pour déterminer des attributs de qualité des échantillons en utilisant une électrode à disque rotatif.

10. Système de mesure et de surveillance intégré à une centrale selon la revendication 9, dans lequel le dispositif analyseur automatisé (34) est configuré pour identifier des contaminants dans des échantillons lorsqu'ils existent, les contaminants comprenant : du Sodium (Na), du Potassium (K), du Lithium (Li), du Vanadium (V), du Magnésium (Mg), du Plomb (Pb), du Nickel (Ni), du Calcium (Ca), du Manganèse (Mn), du Chrome (Cr), du Silicium (Si), du Fer (Fe), de l'Aluminium (Al), du Cuivre (Cu), et du Zinc (Zn).

11. Système de mesure et de surveillance intégré à une centrale selon la revendication 9, comprenant :
un dispositif de conditionnement de signal (84) configuré pour convertir les signaux d'entrée en signaux de sortie interprétables par le système de contrôle distribué ;
dans lequel le dispositif analyseur automatisé est configuré pour fournir les attributs de qualité en tant que signaux d'entrée au dispositif de conditionnement de signaux par l'intermédiaire d'un bus de communication (86).

12. Procédé de mesure et de surveillance intégré à une centrale électrique, comprenant :
la réception, depuis un premier orifice d'échantillonnage (36) disposé dans un premier ensemble de pipelines dans une centrale électrique (10) qui transportent du carburant liquide depuis une source de carburant liquide vers un réservoir de stockage sale (16), d'un échantillon de charge de carburant liquide, l'échantillon de charge (38) représentant le carburant liquide fourni par la source de carburant avant le stockage, le traitement, ou les deux ;
la réception, à partir d'un deuxième orifice d'échantillonnage (44) disposé dans un second ensemble de pipelines dans la centrale électrique (10), d'un échantillon (46) du carburant en aval depuis un composant en aval représentatif du carburant après le traitement, le stockage ou les deux du carburant par le composant en aval (22) ;
l'analyse, par l'intermédiaire d'un dispositif analyseur automatisé (34), de l'échantillon de charge (38) pour déterminer des attributs de qualité de l'échantillon de charge ;
l'analyse, par l'intermédiaire du dispositif analyseur automatisé, de l'échantillon (46) de composant post-aval pour déterminer des attributs de qualité du composant post-aval ; et
la fourniture, à partir du dispositif analyseur automatisé, des attributs de qualité de l'échantillon de charge (38) et des attributs de qualité de l'échantillon (46) de composant post-aval (22) à un système de contrôle distribué (88), permettant au système de contrôle distribué :
d'identifier des anomalies dans le carburant ; et
d'attribuer des anomalies comme une qualité initiale du carburant lorsqu'il est fourni par la source de carburant ou susceptible d'être provoquée par le composant en aval ;
dans lequel le procédé comprend en outre le signalement des conditions de et/ou l'ajustement du fonctionnement de l'équipement dans la centrale électrique (10) en fonction en partie d'une qualité analysée des échantillons de carburant liquide fournis au niveau desdits premier et deuxième orifices d'échantillonnage (36, 44) de la centrale électrique (10).

13. Procédé selon la revendication 13, dans lequel l'analyse de l'échantillon de charge (38) pour déterminer les attributs de qualité de l'échantillon de charge comprend la détection d'une concentration particulaire, d'une concentration en eau, l'identification de contaminants, ou toute combinaison de ceux-ci de l'échantillon de charge.

14. Procédé selon la revendication 13, dans lequel l'analyse de l'échantillon (46) du carburant en aval du composant en aval pour déterminer les attributs de qualité dudit échantillon comprend la détection d'une concentration particulaire, d'une concentration en eau, l'identification de contaminants, ou toute combinaison de ceux-ci dudit échantillon.

15. Procédé selon la revendication 13, dans lequel la fourniture des attributs de qualité de l'échantillon de charge (38) et des attributs de qualité du composant post-aval (22) au système de contrôle distribué (DCS), comprend :
la fourniture des données de qualité et de positionnement concernant le carburant à partir du dispositif analyseur automatisé (34) par l'intermédiaire d'un bus de communication (86) vers le dispositif de conditionnement de signaux (84) ;
la conversion des données de qualité et de positionnement en un signal DCS interprétable par le système de contrôle distribué ; et
la fourniture du signal DCS au système de contrôle distribué (88).

16. Procédé selon la revendication 13, comprenant :
la réception d'au moins un échantillon d'eau (62) et d'au moins un échantillon d'huile lubrifiante (54) à partir des orifices d'eau respectifs (60) et des orifices d'huile lubrifiante (64) ;
l'analyse de l'au moins un échantillon d'eau et de l'au moins un échantillon d'huile pour déterminer des attributs de qualité de l'au moins un échantillon d'eau et de l'au moins un échantillon d'huile ; et
la fourniture des attributs de qualité de l'au moins un échantillon d'eau et de l'au moins un échantillon d'huile au système de contrôle distribué, permettant au système de contrôle distribué :
d'identifier les anomalies dans l'eau de la centrale électrique, l'huile lubrifiante de la centrale électrique, ou les deux ; et
d'identifier un positionnement associé en fonction d'un emplacement où l'au moins un échantillon d'eau et l'au moins un échantillon d'huile ont été émis.

17. Support tangible, non transitoire, lisible par une machine, comprenant des instructions lisibles par une machine, configuré pour amener le système selon la revendication 1 à exécuter les étapes du procédé selon la revendication 12.
